# EUROPEAN PATENT APPLICATION

(11) **EP 1 908 459 A1**
(43) Date of publication of application: **09.04.2008**
(21) Application number: 06781797.3
(22) Date of filing: 27.07.2006
(51) Int. Cl.: A61K 31/122, A23L 1/30, A61P 35/00

(54) **COMPOSITION FOR PREVENTION OF CANCER**

(30) Priority: 28.07.2005 JP 2005218833
(71) Applicant: Kaneka Corporation, Kita-ku Osaka-shi, Osaka 530-8288 (JP); JAPAN as represented by PRESIDENT OF NATIONAL CANCER CENTER, Chuo-ku, Tokyo 104-0045 (JP)
(72) Inventor: WAKABAYASHI, Keiji, c/o NATIONAL CANCER CENTER, Tokyo 104-0045 (JP); TAKAHASHI, Mami, c/o NATIONAL CANCER CENTER, Tokyo 104-0045 (JP); FUJII, Kenji, Kobe-shi, Hyogo 651-1202 (JP); KITANO, Mitsuaki, T akasago-shi, Hyogo 676-0013 (JP)
(74) Representative: Gillet, Raphaëlle
(86) International application number: PCT/JP2006/314886
(87) International publication number: WO 2007/013556

(57) **Abstract**

The present invention has for its subject to obtain a composition for preventing cancer efficient for preventing cancer or preventing cancer recurred after its therapy, or to obtain a pharmaceutical product, food and drink, and cosmetic having a function for preventing cancer.

The present inventors found that a composition containing coenzyme Q has a cancer-preventive effect.

## Description

### TECHNICAL FIELD

The present invention relates to a composition for preventing cancer which can be used for foods and drinks such as health foods or foods with health claims (foods for specified health use, and foods with nutrient function claims), pharmaceutical products, quasi drugs, cosmetics, or the like.

### BACKGROUNG ART

As for cancer therapy, various researches have been made so far. In particular as for drugs for cancer treatment, a lot of researches have been carried out and many products have been developed, and now many drugs are practically used in medical fields. However, at present, a drug which can completely cure cancer has not been developed yet. The problem of drugs for cancer treatment such as anticancer drugs and immunotherapeutic agents currently used arises from the fact that it is difficult to direct drugs to specifically act on cancer cells without affecting normal cells, and from the safety concern that chemically synthesized drugs essentially have. Consequently, at present, the effective dose of the drug is very close to the safe tolerable dose thereof, or the safe tolerable dose is less than the effective dose, and therefore sufficient effectiveness cannot be obtained.

Recently, aside from the therapeutic drug used after development of cancer, in view of inhibiting cancer development, a search for cancer suppressors is considered to be particularly important. If it becomes possible to extract or synthesize an effective cancer suppressor and use it, as a drug or food, for inhibiting cancer development, it can be particularly expected to prevent cancer of high incidence, such as hereditary cancer or occupational cancer. Such drugs or foods are considered to greatly contribute to health maintenance in medical care and in daily life in the future.

Of course, the mechanism to kill existing cancer cells and one to prevent development of cancer are different, however. For example, there is a problem that development of cancer cannot be prevented even if the dosage of drugs for cancer treatment currently used is simply reduced or the usage thereof is simply changed. Therefore, the cancer-preventive effect of a drug is totally unpredictable from its effect for existing cancer, and searches for drugs for preventing cancer are conducted according to the methodology basically different from the one for searching drugs for cancer treatment.

Such drugs for preventing cancer are preferably taken easily in daily life, , and thus a substance or composition showing its effect by oral intake is desired. However, for skin cancer, cosmetics and the like preparations which can be directly applied on the skin are allowable. Furthermore, the most important factor of such substance or composition is to have few side effects on living bodies and high safety because the substance or composition is expected to be taken for a long period of time. However, at present, a composition for preventing cancer, which is highly effective and highly safe and satisfies these conditions, has not so far been found.

Coenzyme Q is a low molecular weight compound found as a biological substance, and it is known to play an important role in biosynthesis of adenosine triphosphate (ATP) as a constituent of mitochondrial electron transport system, and show, as an antioxidative substance, an effect of stabilizing biomembranes and the like effects.

Moreover, in terms of industrial application, coenzyme Q is widely used as a therapeutic agent for congestive heart failure and as a supplement, and its effectiveness is reported not only for cardiac functions, but also for wide range of diseases such as arteriosclerosis, hypertension, diabetes mellitus, and brain diseases. Furthermore, there are also some reports referring the therapeutic effect thereof for breast cancer (Non Patent Document 1) and for other cancer (Patent Document 1 and 2, Non Patent Document 2 and 3), but the preventive effect thereof has not been known at all.
Patent Document 1: Japanese Kokai Publication 2003-135022
Patent Document 2: Japanese Kohyo Publication 2003-532679
Non Patent Document 1: Biochem. Biophys. Res. Commn. (1994)199 1504-1508
Non Patent Document 2: BioFactors (1999) 9 365-370
Non Patent Document 3: Biochem. Biophys. Res. Commn. (1993) 192 241-245

### SUMMARY OF THE INVENTION

The subject of the present invention is to provide a composition suitable for preventing cancer.

Surprisingly, the present inventors found the cancer-preventive effect in coenzyme Q, and achieved the subject of the present invention. Accordingly, the present invention provides the following.
(1) A composition for preventing cancer
   which comprises coenzyme Q represented by the following formula (1): 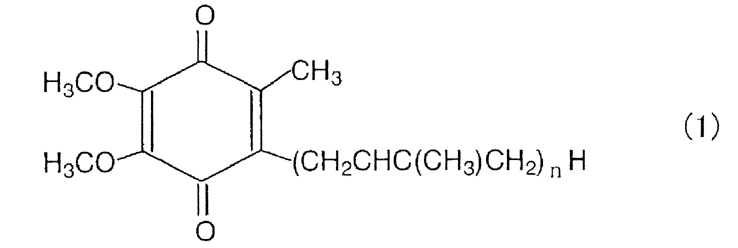 (in the formula, n represents an integer of 1 to 12).
(2) The composition of the above (1),
   wherein coenzyme Q is coenzyme Q₁₀.
(3) A pharmaceutical product or quasi drug
   which comprises the composition of the above (1).
(4) A food or functional food
   which comprises the composition of the above (1).

### DETAILED DESCRIPTION OF THE INVENTION

In the following, embodiments of the present invention are described in detail.

The present invention provides a composition for preventing cancer which comprises coenzyme Q.

Coenzyme Q is represented by the following formula (1) : 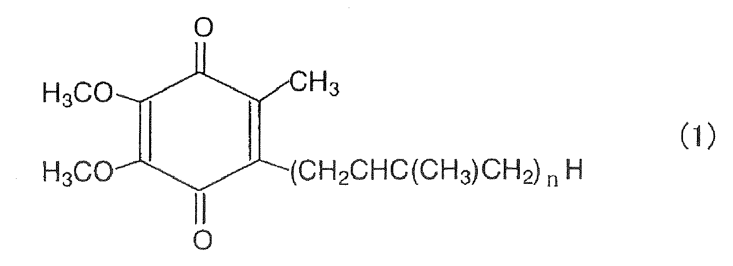 (in the formula, n represents an integer of 1 to 12).

A process for obtaining coenzyme Q is not particularly restricted, and there may be mentioned, for example, conventionally known techniques such as fermentation, chemical synthesis, and extraction from natural products.

As coenzyme Q usable in the present invention, there may be mentioned those having the repeating unit of side chain (n in the formula) of 1 to 12, as represented by the above formula (1). Among them, one having the repeating unit of side chain of 10, i.e., coenzyme Q₁₀ is particularly preferably used.

In the composition of the present invention, in addition to coenzyme Q mentioned above, other materials acceptable in view of practical pharmacy and food hygienic may be arbitrarily added and mixed by the conventional manner. Such materials are not particularly restricted but include, for example, excipients, disintegrants, lubricants, binders, coating agents, colorants, coagulation inhibitors, absorption promoters, solubilizing agents, stabilizers, health food materials, nutritional supplement materials, vitamins, and the like.

The excipients are not particularly restricted but include, for example, white sugar, lactose, glucose, corn starch, mannitol, crystalline cellulose, calcium phosphate, calcium sulfate, and the like.

The disintegrants are not particularly restricted but include, for example, starch, agar, calcium citrate, calcium carbonate, sodium hydrogen carbonate, dextrin, crystalline cellulose, carboxymethylcellulose, tragacanth, and the like.

The lubricants are not particularly restricted but include, for example, talc, magnesium stearate, polyethylene glycol, silica, hydrogenated vegetable oils, and the like.

The binders are not particularly restricted but include, for example, ethylcellulose, methylcellulose, hydroxypropymethyllcellulose, tragacanth, shellac, gelatin, gum arabic, polyvinylpyrrolidone, polyvinyl alcohol, polyacrylic acid, polymethacrylic acid, sorbitol, and the like.

The coating agents are not particularly restricted but include gum arabic, Opadry, prunella spike, castor wax, carboxyvinyl polymers, carmellose, hydrous silicon dioxide, magnesium silicate, vinyl acetate resins, stearic acid, cetanol, hydroxypropylmethylcellulose, and the like.

The colorants are not particularly restricted but those authorized to be added to drugs and/or foods, and the like can be used, for example.

The coagulation inhibitors are not particularly restricted but include, for example, stearic acid, talc, light silicic anhydride, hydrous silicon dioxide, and the like.

The absorption promoters are not particularly restricted but include, for example, higher alcohols, higher fatty acids, surfactants such as glycerol fatty acid esters, and the like.

The solubilizing agents are not particularly restricted but include, for example, organic acids such as fumaric acid, succinic acid, malic acid, and the like.

The stabilizers are not particularly restricted but include, for example, benzoic acid, sodium benzoate, ethyl parahydroxybenoate, and the like.

The health food materials are not particularly restricted but include Kampo (Chinese) medicines (e.g. Irei-to (crude drug extract for treating stomach disorder, etc.), Unkei-to (crude drug extract for warming body, etc.), Unsei-in (crude drug extract for promoting blood circulation, etc.), Ogi-kenchu-to (extract of Astragalus membranaceus, etc.), Oren-gedoku-to (crude drug extract for reducing fever, inflammation, etc.), Oren-to (extract of Coptis is, etc.), Kakkon-to (extract of Puerariae radix, etc.), Kami-kihi-to (crude drug extract for treating anemia, insomnia, neurosis, etc.), Kami-shoyo-san (crude drug extract for treating menstrual and climacteric disorder, etc.), Kam-baku-taiso-to (crude drug extract for treating night cry, convulsion, etc.), Kikyo-to (extract of Platycodon grandiflorum, etc.), Kihi-to (crude drug extract for treating anemia, insomnia, etc.), Kumi-binro-to (extract of nine crude drug species, e.g. Livistona chinesis var. subglobosa), Keigai-rengyo-to (crude drug extract for treating chronic paranasal sinusitis, acne, etc.), Keishi-ka-shakuyaku-daio-to (crude drug extract for treating stomach disorder, etc.), Keishi-ka-shakuyaku-to (extract of Cinnamomi cortex, Chinese peony, etc.), Keishi-ka-ryukotsu-borei-to (extract of Cinnamomi cortex, Ostrea gigas, etc.), Keishi-to (extract of Cinnamomi cortex, etc.), Keishi-ninjin-to (extract of Cinnamomi cortex, gensing, etc.), Keishi-bukuryo-gan (extract of Cinnamomi cortex, Poria cocos, etc.), Keihi-to (crude drug extract for treating digestive trouble, diarrhea, etc.), Koso-san (extract of Cyperus rotundus, etc.), Goko-to (crude drug extract for treating cough, asthma, etc.), Goshaku-san (crude drug extract for treating blood and water circulation, etc.), Gosha-jinki-gan (crude drug extract for improving body function, etc.), Gorin-san (crude drug extract for treating frequent urination, miction pain, etc.), Saikan-to (extract of Bupleurum chinense, etc.), Saiko-ka-ryukotsu-borei-to (extract of Bupleurum chinense, Ostrea gigas, etc.), Saiko-keishi-kankyo-to (extract of Bupleurum chinense, Cinnamomi cortex, Zingiber officinale, etc.), Sazko-keishi-to (extract of Bupleurum chinense, Cinnamomi cortex, etc.), Saiko-seikan-to (extract of Bupleurum chinense, Scutellaria baicalensis Georgi, etc.), Saiboku-to (extract of Bupleurum chinense, Scutellaria baicalensis Georgi, Pinellia ternate, etc.), Sairei-to (crude drug extract for reducing inflammation, improving water circulation, etc.), Sansonzn-to (extract of Zizyphus jujuba, etc.), Jiin-koka-to (crude drug extract for tempering cough), Shigyaku-san (extract of Bupleurum chinense, etc.), Shikunshi-to (crude drug extract for improving stomach function, etc.), Shimotsu-to (extract of four crude drug species e.g. Angelica sinensis, etc.), Shakanzo-to (crude drug extract for tempering palpitation and breath shortness, etc.), Shakuyaku-kanzo-to (extract of Paeonia lactiflora and Glycyrrhiza uralensis), Juzen-taiho-to (crude drug extract for recovering vitality and energy), Jumi-haidoku-to (extract of ten crude drug species and used for dermatitis, etc.), Sho-kenchu-to (crude drug extract for improving stomach function, etc.), Sho-saiko-to (crude drug extract for protecting liver, etc.), Sho-seiryu-to (crude drug extract for treating allergic rhinitis, asthma, etc.), Shofu-san (crude drug extract for treating eczema), Shin'i-seihai-to (extract of Magnolia kobus, etc.), Shimpi-to (extract of Ephedra sinica, etc.), Shimbu-to (crude drug extract for warming body to improve body function, etc.), Seijo-bofu-to (crude drug extract for treating acne), Seisho-ekki-to (crude drug extract for treating summer weariness, etc.), Seishin-renshi-in (crude drug extract for treating urination disorder, etc.), Seihai-to (extract of Magnolia praecocissima, etc.), Sokei-kakketsu-to (crude drug extract for reducing nerve pain, backache, etc.), Daio-kanzo-to (extract of Rheum tanguticum and Glycyrrhiza uralensis), Daio-botampi-to (extract of Rheum tanguticum, Paeonia suffruticosa, etc.), Dai-kenchu-to (crude drug extract for reducing stomachache, etc.), Dai-saiko-to (extract of Bupleurum chinense, Scutellaria baicalensis Georgi, etc.), Dai-saiko-to-kyo-daio (extract of Bupleurum chinense, Scutellaria baicalensis Georgi, Pinellia ternate, etc.), Dai-joki-to (crude drug extract for treating constipation, etc.), Dai-bofu-to (extract of Ledebouriella seseloides, etc.), Ji-daboku-ippo (crude drug extract for treating bruise), Choi-joki-to (crude drug extract for loosening the bowels, etc.), Choto-san (extract of Uncaria rhynchophylla, etc.), Choyo-to (crude drug extract for reducing hypogastric region pain), Chorei-to (extract of Polyporus umbellatus, etc.), Chorei-to-go-shimotsu-to (extract of Polyporus umbellatus, etc.), Tsu-do-san (crude drug extract for treating menstrual disorder, menstrual cramps, etc.), Tokaku-joki-to (crude drug extract for treating constipation, menstrual disorder, etc.), Toki-inshi(crude drug extract for treating eczema, dry skin itch, etc.), Toki-kenchu-to (extract of Angelica sinensis, Cinnamomi Cortex, Paeonia lactiflora, etc.), Toki-shakuyaku-san (extract of Angelica sinensis, Paeonia lactiflora, etc.), Toki-to (extract of Angelica sinensis, etc.), Nichin-to (crude drug extract for reducing nausea, vomiting, etc.), Nyoshin-san (crude drug extract for treating flash, dizziness, etc.), Ninjin-to (extract of gensing, etc.), Ninjin-yoei-to (extract of gensing, Astragalus membranaceus, etc.), Haino-san-kyu-to (crude drug extract for treating skin tumor, etc.), Bakumondo-to (extract of Ophiopogon japonicus, etc.), Hachimi-jio-gan (extract of eight crude drug species e.g. Rehmannia glutinosa, etc.), Hange-koboku-to (extract of Pinellia ternate, Magnolia officinalis, etc.), Hange-shashin-to (extract of Pinellia ternate, etc.), Byakko-ka-ninjin-to (extract of Gypsum, etc.), Bukuryo-in (extract of Poria cocos, etc.), Bukuryo-in-go-hange-koboku-to (crude drug extract for reducing emesis of pregnancy, etc.), Heii-san (crude drug extract for treating heavy stomach, etc.), Boiogi-to (extract of Aristolochia fangchi, Astragalus membranaceus, etc.), Bofu-tsusho-san (extract of Ledebouriella seseloides, etc.), Hochu-ekki-to (crude drug extract for improving stomach function to invigorate, etc.), Mao-to (extract of Ephedra sinica, etc.), Mao-bushisaishin-to (extract of Ephedra sinica, Aconitum carmichaeri, etc.), Ma-kyo-kan-seki-to (extract of Ephedra sinica, Prunus armeniaca, etc.), Mashinin-gan (extract of Cannabis sativa, etc.), Moku-boi-to (extract of Cocculus trilobus, etc.), Yoku-kan-san (crude drug extract for reducing nerve excitement, etc.), Yoku-kan-san-ka-chimpi-hange (crude drug extract for reducing nerve excitement, etc.), Rikkunshi-to (crude drug extract for reducing heavy stomach, etc.), Rikko-san (crude drug extract for reducing toothache, etc.), Ryutan-shakan-to (extract of Gentiana scabra, etc.), Ryo-kan-kyo-mi-shin-ge-nin-to (crude drug extract for controlling cough and clearing throat, etc.), Rokumi-gan (extract of six crude drug species and used for improving body function, etc.), and the like), tea leaves (e.g. green tea, Japanese tea mixed with roasted rice, powdered green tea, green tea of middle grade, toasted tea, roasted tea, jasmine tea, oolong tea, tea, black tea, flower tea, blue tea, white tea, etc.), herbs (e.g. Italian parsley, elecampane, olive, oregano, cardoon, chamomile, curry plant, catnip, caraway, Christmas rose, crimson clover, cornflower, common mallow, salad burnet, santolina, cinnamon, jasmine, stevia, sage, linden (lime), scented geranium, St.-John's-wort, soapwort, Solomon's seal, thyme, tansy, chervil, chive, nasturtium, nutmeg, basil, honeysuckle, hyssop, flax, fennel, foxglove, black hollyhock, French marigold, betony, heliotrope, bergamot, hemp agrimony, rue, pot marigold, borage, white horehound, myrtle, mullein, marjoram, mint, yarrow, lavender, Lady's bedstraw, lemongrass, lemon verbena, lemon balm, rose, rosemary, rocket, wild strawberry, wild pansy, forget-me-not, etc.), propolis, gingko leaves, chlorophyll juice, extracts from these, and the like.

The nutritional supplement materials are not particularly restricted but include amino acids, metal ions, proteins, saccharides, fatty acids, yeast extracts, vegetable extracts, fish meat extracts, fruits, fruit extracts, and the like.

The vitamins are not particularly restricted but include, for example, vitamin A, vitamin B, vitamin C, vitamin D, vitamin E, vitamin K, derivatives derived from these, and the like.

The composition of the invention can be used in pharmaceutical products, quasi drugs, foods, functional foods, food materials, cosmetic materials, or the like. The functional foods cited herein refer to products for maintaining or improving health by oral intake of products other than pharmaceutical products, such as supplements, foods for specified health uses, health foods, or dietary supplements.

With the composition for preventing cancer of the invention, a pharmaceutical preparation or other active ingredients intended for oral intake can be used without any restrictions.

The dosage form of the composition of the invention is not particularly restricted and may be, for example, powders, granules producible by adding a binder, powders coated with a coating agent, and capsule preparations producible by filling powders, granules or coated powders into capsules. Also employable are soft capsule preparations producible by adding natural oil, higher fatty acid oil, a higher fatty acid monoglyceride, a surfactant, or mixture thereof, etc. and filling that in its oily state into capsules. In this case, as the capsule, gelatin-based ones or ones based on other soluble polymer substances than gelatin can be used, for example. Also, microcapsules are included in such capsules. Furthermore, liquid form is also employable which is producible by preparing an emulsion using a surfactant, an oil and fat, and the like.

These compositions may contain an antioxidant. The antioxidant includes citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α-lipoic acid and derivatives thereof, pycnogenol, flavangenol, superoxide dismutase (SOD), glutathione peroxidase, glutathione-S-transferase, glutathione reductase, catalase, ascorbic acid peroxidase, mixtures of these, and the like.

In cases where the composition for preventing cancer according to the invention takes a food form, the food form is not particularly restricted but includes edible fat and oil compositions, cooking oils, spray oils, butters, margarines, shortenings, whipped creams, condensed milks, whiteners, dressings, pickling solutions, breads, cakes, pies, cookies, Japanese sweets, snack foods, fried cookies/sweets, chocolates, chocolate candies, rice crackers, roux, sauces, drippings, toppings, ice cream/iced sherbet, noodles, bakery mixes, fried foods, processed meat products, fish paste-based products, frozen entrees, frozen stock farm products, frozen agricultural products, other frozen foods, boiled rice products, jams, cheeses, cheese foods, cheese-like foods, gums, candies, fermented milk products, canned foods, drinks and beverages, and the like.

The composition for preventing cancer of the invention can also be parenterally administered. As a parenteral administration form, there may be mentioned one obtainable by dissolving, or mixing and dispersing coenzyme Q in an appropriate base to prepare cream, paste, jelly, gel, emulsion, and liquid (e.g. ointment, liniment, lotion or spray); one obtainable by dissolving, or mixing and dispersing the above-mentioned drug in a base, and flatting the resultant on a support (e.g. catalpasm); one obtainable by dissolving, or mixing and dispersing coenzyme Q in an adhesive, and flatting the resultant on a support (e.g. plaster, tape); eye drop, suppository, injection, and the like.

In the case where the composition for preventing cancer of the invention is applied to the skin, the above composition may further contain an ingredient activating the skin, for example, collagen, hyaluronic acid, mucin, ceramide, squalene, squalane, and the like, or an agent for promoting percutaneous absorption.

The composition for preventing cancer of the invention can also be used as a cosmetic composition or a skin healthcare composition. As its specific application, there may be mentioned but not limited to facial wash, eye cream, eye shadow, cream, emulsion, skin toner, perfume, face powder, cosmetic oil, paste perfume, powder, face mask, shaving cream, shaving toner, tanning oil, sunscreen oil, tanning lotion, sunscreen lotion, nail cream, nail enamel, bath product, blusher, mascara, lipstick, lip cream, eye liner, deodorant, cologne, and the like.

In such cases, the above composition may contain cosmetic adjuvants which are conventionally used for cosmetic or skin healthcare composition. As such component, there may be mentioned, for example, preservatives, disinfectants, perfumes, foaming inhibitors, colorants, pigments having a coloring action, thickeners, surfactants, emulsifiers, softening agents, humidifying agents and/or moisturizers, fats, oils, waxes, alcohols, polyols, polymers, bubble stabilizers, electrolytes, organic solvents, silicone derivatives, etc.

The content of coenzyme Q, dosage form, preservation method and preservation form in producing the coenzyme Q-containing composition of the invention can be appropriately determined according to the application of the composition, such as pharmaceutical products, quasi drugs, health foods, foods, functional foods, cosmetics, animal drugs, and animal feed.

However, the content of coenzyme Q in the composition of the invention is desirably 0.001 to 20% by weight, and more desirably 0.005 to 10% by weight.

The term "preventing cancer" cited in this specification refers to inhibition of development of a cancer cell and suppression of growth of a cancer cell once developed. The species of tumors to be inhibited by the composition for preventing cancer of the invention is not restricted. That is, epithelial tumors and nonepithelial tumors are both included, and further whether the tumors are benign or malignant is not considered. For example, tumors such as epithelial tumors, nonepithelial tumors, nervous system tumors, bone neoplasms, and lymphoid tumors developed in various organs of the body such as the stomach, intestine, lung, liver, kidney, pancreas, gallbladder, uterus, ovary, testis, prostate or brain are included.

When the composition for preventing cancer of the invention is administered as a pharmaceutical product or quasi drug, or when it is taken as a food or drink, the aim for administration may include prevention of inheritance cancer, prevention of cancer resulting from environment or lifestyle, prevention of recurrence and metastasis of cancer after tumor resection, or as one option of cancer therapy. More specifically, there may be mentioned the cases where a person is recognized to have high possibility of developing cancer in the future by gene diagnosis and therefore, before onset, takes the composition for preventing cancer of the invention preventively, where a person who fears hereditarily developing cancer takes the composition preventively, where a person who has a smoking habit or is exposed to radioactive material at work takes the composition preventively, or where a cancer patient takes the composition on behalf of or in parallel with an anticancer drug for chemotherapy, but there is no limitation.

The dosage of the composition for preventing cancer of the invention is not particularly restricted, but is preferably 30 mg/day to 600 mg/day per adult in terms of the weight of coenzyme Q.

### (Effect of the invention)

According to the present invention, it becomes possible to provide a composition for preventing cancer which can be used for foods and drinks such as foods, health foods, and foods with health claims (foods for specified health use, and foods with nutrient function claims), pharmaceutical products, quasi drugs, cosmetics, or the like.

### BEST MODE FOR CARRYING OUT THE INVENTION

The following examples illustrate the present invention in further detail. These examples are, however, by no means limitative of the scope of the invention.

### <Example 1> Cancer prevention evaluation

6-week-old male F344 rats were divided into 3 groups (2 treated groups and 1 control group; each group consisted of 9 rats) and a colon carcinogen azoxymethane (AOM) was injected subcutaneously at the dose of 15 mg/kg body weight once a week over two weeks. From the day before injection of AOM, to the treated groups, coenzyme Q₁₀ (Kaneka Q₁₀, Kaneka Corporation) was fed together with feed at the concentration of 200 or 500 ppm, respectively. 4 weeks later, aberrant crypt foci (ACF), which is a precancerous change in colon cancer, was analyzed. After the rats were sacrificed, the colons (from the cecum to rectum) were enucleated and fixed using a 10% neutral buffered formalin. After the fixation, the colons were washed with a phosphate buffered-saline and stained with a 0.2% methylene blue-containing phosphate buffered-saline. Then, the number and size of ACF were measured using a microscope. As a result, the number of formation of ACF in the coenzyme Q₁₀-treated group (200ppm, 500ppm) decreased to 76.6% or 68.0% (p<0.01), respectively, compared with the control group. On the other hand, size difference in ACF was not recognized between the control group and coenzyme Q₁₀-treated groups. As observed above, it was made clear that coenzyme Q₁₀ has an effect for inhibiting the initial stage of AOM-induced formation of colon ACF, which is a precancerous change of colon cancer.

### <Preparation Example 1> (Powder preparation)

Coenzyme Q₁₀ was dissolved in propanol and then allowed to be adsorbed on microcrystalline cellulose, followed by drying under reduced pressure. The dried product was mixed with corn starch to give a powder preparation. The formulation is as follows.

| | |
|---|---|
| Coenzyme Q₁₀ | 10 parts by weight |
| Microcrystalline cellulose | 40 parts by weight |
| Corn starch | 55 parts by weight |

### <Preparation Example 2> (Capsules)

A powder preparation was prepared in the same manner as in Preparation Example 1 and filled into gelatin capsules in the conventional manner. The formulation is as follows.

| | |
|---|---|
| Coenzyme Q₁₀ | 20 parts by weight |
| Microcrystalline cellulose | 30 parts by weight |
| Corn starch | 20 parts by weight |
| Lactose | 25 parts by weight |
| Magnesium stearate | 3 parts by weight |
| Polyvinylpyrrolidone | 2 parts by weight |

### <Preparation Example 3> (Soft capsules)

Coenzyme Q₁₀ melted at 50°C was added to and dissolved in corn oil warmed to the same temperature. Soft capsules were filled with that solution in the conventional manner. The formulation is as follows.

| | |
|---|---|
| Coenzyme Q₁₀ | 50 parts by weight |
| Corn oil | 350 parts by weight |

### <Preparation Example 4> (Tablets)

Coenzyme Q₁₀ was dissolved in propanol and then allowed to be adsorbed on microcrystalline cellulose, followed by drying under reduced pressure. The dried product was mixed with corn starch, lactose, carboxymethylcellulose and magnesium stearate and, after addition of an aqueous solution of polyvinylpyrrolidone as a binder, the mixture was granulated in the conventional manner. Talc, as a lubricant, was added to the granules and, after mixing up, the mixture was made into tablets.

| | |
|---|---|
| Coenzyme Q₁₀ | 20 parts by weight |
| Corn starch | 25 parts by weight |
| Lactose | 15 parts by weight |
| Carboxymethyl cellulose | 10 parts by weight |
| Microcrystalline cellulose | 40 parts by weight |
| Polyvinylpyrrolidone | 5 parts by weight |
| Magnesium stearate | 3 parts by weight |
| Talc | 10 parts by weight |

### <Preparation Example 5> (Hydrophilic ointment)

A hydrophilic ointment containing coenzyme Q₁₀ was prepared by a known process according to the following composition.

| | |
|---|---|
| Hydrophilic ointment | 96% by weight |
| Coenzyme Q₁₀ | 1% by weight |
| Ascorbic acid stearic acid ester | 3% by weight |

### <Preparation Example 6> (W/O emulsion)

A W/O emulsion containing coenzyme Q₁₀ was prepared by a known process according to the following composition.

| | |
|---|---|
| Polyoxyethtleneglycol sorbitan fatty acid ester | 3.60% by weight |
| Polyoxyethtlene fatty acid ester | 1.40% by weight |
| Stearyl alcohol | 2.00% by weight |
| Mineral oil, GP9 | 20.00% by weight |
| Paraben mixture | appropriately |
| Magnesium sulfate (MgSO₄·7H₂O) | 0.70% by weight |
| Coenzyme Q₁₀ | 1.00% by weight |
| Calcium chloride (CaCl₂) | 0.85% by weight |
| Vitamin E | 1.00% by weight |
| Deionized water (the level of addition is to be adjusted such that the total amount is 100.00% by weight) | |

## Claims

1. A composition for preventing cancer
which comprises coenzyme Q represented by the following formula (1): 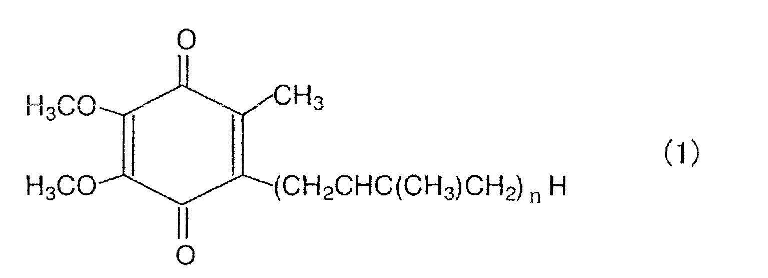 (in the formula, n represents an integer of 1 to 12).

2. The composition according to Claim 1,
wherein coenzyme Q is coenzyme Q₁₀.

3. A pharmaceutical product or quasi drug
which comprises the composition according to Claim 1.

4. A food or functional food
which comprises the composition according to Claim 1.
